# EUROPEAN PATENT SPECIFICATION

(11) **EP 2 304 428 B1**
(45) Date of publication and mention of the grant of the patent: **17.04.2019**
(21) Application number: 09773080.8
(22) Date of filing: 02.07.2009
(51) Int. Cl.: G01N 33/02

(54) **SYSTEM FOR ENVIRONMENT DIAGNOSTIC AND MONITORING AND SELF-CONTROL OF FOOD CHAIN**
SYSTEM ZUR UMWELTDIAGNOSTIK UND BEOBACHTUNG UND SELBSTKONTROLLE DER NAHRUNGSKETTE
SYSTÈME POUR LE DIAGNOSTIC ET LA SURVEILLANCE DE L`ENVIRONNEMENT ET L`AUTOPROTECTION DE LA CHAÎNE ALIMENTAIRE

(30) Priority: 02.07.2008 IT RM20080362
(43) Date of publication of application: 06.04.2011
(73) Proprietor: Istituto Superiore di Sanità, 00161 Roma (IT)
(72) Inventor: FRAZZOLI Chiara, 00199 ROMA (IT); MANTOVANI Alberto, 00161 ROMA (IT); CAMPANELLA, Luigi, 00141 ROMA (IT); DRAGONE, Roberto, 00186 ROMA (IT)
(74) Representative: Santi, Filippo
(86) International application number: PCT/IT2009/000293
(87) International publication number: WO 2010/001432

(56) References cited:
- WO-A-00/39577
- US-A1- 2002 116 155
- US-B2- 7 393 380
- CHANG K S ET AL: "Series quartz crystal sensor for remote bacteria population monitoring in raw milk via the Internet" BIOSENSORS AND BIOELECTRONICS, ELSEVIER BV, NL, vol. 21, no. 8, 15 February 2006 (2006-02-15), pages 1581-1590, XP024961450 ISSN: 0956-5663 [retrieved on 2006-02-15]
- EKLOV TOMAS ET AL: "Monitoring sausage fermentation using an electronic nose" JOURNAL OF THE SCIENCE OF FOOD AND AGRICULTURE, vol. 76, no. 4, April 1998 (1998-04), pages 525-532, XP002557358 ISSN: 0022-5142
- ILARIA PALCHETTI ET AL: "Electroanalytical biosensors and their potential for food pathogen and toxin detection" ANALYTICAL AND BIOANALYTICAL CHEMISTRY, SPRINGER, BERLIN, DE, vol. 391, no. 2, 17 February 2008 (2008-02-17), pages 455-471, XP019621283 ISSN: 1618-2650
- JOST BORCHERDING: "Ten Years of Practical Experience with the Dreissena-Monitor, a Biological Early Warning System for Continuous Water Quality Monitoring", HYDROBIOLOGIA, KLUWER ACADEMIC PUBLISHERS, DO, vol. 556, no. 1, 1 February 2006 (2006-02-01), pages 417-426, XP019245614, ISSN: 1573-5117
- P Schmitz ET AL: "DEVELOPMENT, TESTING AND IMPLEMENTATION OF AUTOMATED BIOTESTS FOR THE MONITORING OF THE RIVER RHINE, DEMONSTRATED BY BACTERIA AND ALGAE TESTS", Wat. Sci. Tech, 1 January 1994 (1994-01-01), pages 215-221, XP055346898, Retrieved from the Internet: URL:http://wst.iwaponline.com/content/ppiw awst/29/3/215.full.pdf [retrieved on 2017-02-16]
- Diego L. García-González ET AL: "Virgin Olive Oil Quality Classification Combining Neural Network and MOS Sensors", Journal of Agricultural and Food Chemistry, vol. 51, no. 12, 1 June 2003 (2003-06-01), pages 3515-3519, XP055347088, US ISSN: 0021-8561, DOI: 10.1021/jf021217a

## Description

The present invention relates to a system (BEST - Integrated Toxicity (Bio) Sensors' System for hazard analysis and management in the food chain and the environment) for simultaneous and continuous identification, control, monitoring, of healthiness (with series of grids of toxicity indexes) in a food productive chain and/or environment and relevant methods for data detection and analysis, signaling and quick managing of hazards.

More specifically, the invention concerns an integrated system permitting individuating and monitoring possible anomalous variations of toxicity indexes in the control critical points (CCP) and other points where a particular attention of the whole agro-zootechnical productive chain (*Process Analytical Technology,* or PAT), as well as in environmental compartments, particularly studied and realized with reliability, saving costs, ease of interpretation and use, high productivity, automation, eco-compatibility features (even by solar energy supply), with reduced dimensions in order to have a increased portability, such as to make it usable in the routine.

In the following, specification will be mainly addressed to the application for actuation of self-control of the toxicological risk in the agro-zootechnical chain, but it is well evident that the same must not be considered limited to this specific use, being it possible identification, control, monitoring and managing in other productive fields or with environmental toxicity indexes.

According to the HACCP system and self-control, healthiness of food, including safety under the toxicology point of view, must be ensured all along the food productive chain. By the term "food chain" it is meant the entire path determining production of a foodstuff, from raw materials to the final consumer. Food chain safety and healthiness, and product quality defense is responsibility directly involving all the protagonist of the food production system.

Therefore, food companies - regardless from their dimensions and role they have in the food chain, are in any case obliged to apply self-control systems in the manufacturing plants and in breedings in order to ensure healthiness of their production in each step, from primary production (such as Agricola production, zootechny, fishing) to preparation, transformation, conservation, selling and administering food.

Nowadays, always more felt are tracking and monitoring needing in any kind of the food chains, and in each moment and production or transformation stage, and thus of the possible contrary long-medium term effects for health associated with environmental quality, unwished substances and animal welfare.

In order to guarantee the high quality of foods put on the market, instruments are presently available which are widely used in Europe, such as Good Production Practice and HACCP system. Said instruments, suitable to examine food chains under a hygienic-sanitary point of view, can be employed only in few cases in order to prevent risks of microbiological contamination prevention and in some cases to verify organoleptic parameters. In order to identify, evaluate, control, monitor and managing chemical/toxicology risk, apart from analytical determinations generally addressed to measure a limited number of substances of the finished product, no device is presently available to be used in the chain and by not-specialized operators, and thus for real self-control on this field.

Strict correlation between agro-zootechnical and environment productive chains makes it important availability of new devices both for diagnostic along the food productive chain and for environment quality, including the agro-zootechnical field (for example eco-toxicity, bio-remediation check and environmental impact check of the same food chains, e.g. potential of eco-toxicity of breeding wastes).

Innovation of the self-control methods can no more be delayed. New concept of food safety involves that HACCP strategies are prepared to include and evaluate, besides acute effects, also medium and long term risks; therefore, it is important having new instruments capable putting into evidence indicators also of not immediate risks.

Furthermore, European Commission recommends the use, valorization and optimization of already available studies and products (European Strategy for Environment and Health), extension of control from end product to manufacturing processes (EU White Book for food safety "From farm to folk"), and strengthening of traceability in food chain by quick, automatic systems, high productivity, economic, reliable, that can be simply interpreted by the operator and that can be used in the productive routine.

Therefore, multiparameter control systems are always more required that can be used on matrixes and with different situations, e.g. on food and feed productive chains, depuration processes (e.g. waste waters, process wastes, ecc.), analyses of agro-zootechnical practice impacts, but also for detection of alteration and reversibility of conformity parameters of food and environment. Furthermore, an essential aspect to have a valid valuation and control system is possibility of early monitoring and managing hazards.

Obviously, present sophisticated systems for instrument analyses permit detection of specific contaminant substances within foodstuffs. However, just for complexity and vastness reached by production and food chain, as well as continuous rising of new "alarms" and of new substances which are noxious for health, it is often difficult permitting early individuating unhealthiness of a product, of one of its components, thus preventing not adequacy of the finished product to the commercialization.

Furthermore, real contamination cases often are more than the presence of a single not wished substance. It must be pointed out that environmental and food matrixes are complex and can cause the so called mixture effect, comprising both not wished and protective substances.

Main technical problem of those operating in this sector wishing controlling and demonstrating healthiness of their food productive chain is that no system is commercially available which is suitable to toxicology self-control in chain, also to address possible subsequent and more expensive chemical analyses on positive samples: this would permit saving resources and time, ensuring a higher product safety degree and thus both preventing not adequacy to commercialization of the finished product and promoting commercialization.

The relevant prior art includes the patent application WO0039577, which describes system for detection of contaminants. Furthermore, relevant prior art comprises patent applications US 7393380 B2 and US 2002/116155 A.

A related system for environmental diagnostics and monitoring is disclosed in JOST BORCHERDING: "Ten Years of Practical Experience with the Dreissena-Monitor, a Biological Early Warning System for Continuous Water Quality Monitoring",HYDROBIOLOGIA, KLUWER ACADEMIC PUBLISHERS, vol. 556, no. 1, 1 February 2006 (2006-02-01), pages 417-426.

Object of the present invention is that of realizing in the food chain and/or in environment a self-control in the toxicological field *in situ* using a multiparameter integrated system (BEST - Integrated Toxicity (Bio) Sensors' System for hazard analysis and management in the food chain and the environment).

The present invention relates to a system for environmental diagnostics and monitoring according to independent claim 1. Further aspects of the invention are laid out by the accompanied dependent claims. It is therefore specific object of the present invention a system for environmental diagnostic and monitoring and toxicologcal self-control in the food chain, including primary production, comprising: a detecting unit having a plurality of independently selectable probes, each one suitable to detect a respective parameter for valuing the toxicity in one or more Critical Control Points (CCP) and/or points of particular attention of a food chain and/or of a environmental compartment thereby generating a suitable signal, and a data collecting module, connected to said probes, suitable to store the so detected data; and a data processing unit, connectable to said data collecting module for acquiring said detected data of said probes and provided with user interface means, said data processing unit being suitable to determine and indicate by said interface means through control charts and/or other diagrams, at least an alert threshold and at least a intervention threshold for each parameter detected by said selected probes, so as to compare the detected data of said probes to said thresholds to provide for a warning indication in said Critical Control Points (CCP) and/or points of particular attention of said food chain and/or said environmental compartments.

Always according to the invention, said system comprises a plurality of electronic boards, each one connected to one respective probe and to said data collecting module, and suitable to carry out a preprocessing of the signal of the respective probe.

Still according to the invention, said detection unit can be supplied by electric supply, or by a battery, or by eco compatible sources, such as solar energy.

Furthermore according to the invention said parameters can be of biological and/or chemical and/or physical type and said system is suitable to carry out diagnostic, environment monitoring and toxicological self-control in the food chain, including primary production, by early identification, control, monitoring and managing of anomalous variations of a grid of integrated index, such as bio-markers of total exposure or of efficient dose, aspecific toxicity, specific toxicity, secondary and support indexes, with respect to a variation range which is considered normal.

Advantageously, according to the invention, said probes can be of the electric/electrochemical/optic type, preferably of the type coupled with microorganisms, said organisms being whole or part of microorganism, facultative aerobe, such as leaven cells, or obliged, such as human cellular lines or even of the photosynthetic or chemo-luminescent type for valuation of the integral toxicity index by measure of cellular respiration and/or photosynthetic activity and/or of chemo-luminescence and/or of the type coupled with general indicators of oxidoreductase enzymatic activity and/or DNA and/or immunosensors.

Always according to the invention said signal generated by said probes can be of analogical and/or digital type.

Still according to the invention said probes can be suitable to measure further indexes, such as glucose and/or the oxygen biological demand (BOD) and/or secretion enzymes and/or active concentrations of pesticides and antibiotic and/or presence of ions.

Furthermore according to the invention, said probes can be suitable to measuring pH and/or temperature and/or conductivity and/or redox potentiality and/or O₂/CO₂ and/or Chemical Oxygen Demand (COD).

Advantageously according to the invention said probes are modular and can be suitable to the specific problems identified in a specific chain and/or measure situation.

Always according to the invention, said probes are maintained in a liquid fluid thermostated environment or in a climatised environment, at a controlled temperature, with monitoring and registration of the operating temperature.

Still according to the invention said data collecting module can be connected with said data processing unit by a serial interface for intranet and/or by wireless connection and/or by intranet network and/or by internet network, to transmit data from said detections, said data processing unit can be connected to a plurality of said data collecting module.

Advantageously, according to the invention, said system can comprise an acoustic and/or optic warning device suitable to emit a signal in case at least one detection of one of said probes goes beyond the respective warning or alert threshold.

Always according to the invention, said data processing unit is configured to carry out a multivariate analysis of the values of data detected by said probes.

Still according to the invention, said data processing unit can be based on neural networks.

Always according to the invention, said system can comprise a menu, by which the parameters and the respective probes are preselected or preselectable for specific foods or for specific critical control points (CCP) and/or points of particular attention in the food chain and/or in environment.

Still according to the invention, said interface means comprises a monitor.

Advantageously, according to the invention, said system can communicate by wireless transceiving means or by intranet or internet network, with further systems, each one of said systems being applied in different environmental compartments and/or different Control Critical Points (CCP) and/or points of particular attention along the different branches of a food chain or environment, including primary production.

A method for analyzing data, and quick signaling and managing of hazards due to anomalous variations of biological activities by toxicity indexes in the food chain and/or in environment by a system as defined in the above, comprises the following steps for realizing control charts and/or other diagrams:
- carrying out a plurality of measures of one or more parameters by means of corresponding probes;
- setting an expected measurement value of each one of said parameters;
- calculating a variability interval or physiological variation for each one of said plurality of measures for each one of said parameters;
- calculating for one or more parameters at least one alert indication threshold as a function of said physiological variation;
- calculating for one or more parameters at least one intervention indication threshold in function of said physiological variation, suitable to provide an intervention indication for controlling food healthiness and/or toxicity indexes in the food chain and/or in the environment.

Always according to the invention, expected value of a parameters is preset and/or established by the practice in the specific application field and/or derived by the measurements.

Still according to the invention, the expected value of a parameter is equal to the average of the measurements carried out, said physiological variation being variability interval of said measurements of said parameters and robustness and precision of said control chart increasing consistency of said expected value and of said physiological variation following the input of data from food chain.

Advantageously according to the invention, said alert indication threshold can be equal to twice the physiological variation.

Furthermore, according to the invention, said intervention indication threshold can be equal to three times the physiological variation.

The method can comprise the following further step:
- generating an alarm signal in case detection of at least one parameter passes two consecutive times the respective intervention indication threshold and/or at least once the relevant intervention indication threshold.

Still according to the invention, said method can comprise the following further step:
- displaying, on said interface means, one control chart for each detected parameter, suitable to display the respective detected data, the expected value, said at least one alert indication threshold, said at least an intervention indication threshold, and sample - acquisition errors.

Preferably, said method can comprise the following further step of data evaluation and analyses:
- carrying out a multivariate analysis of said measurements with respect to said selected parameters, even by development of a neural network.

The present invention will be now described for illustrative but not limitative purposes according to its preferred embodiments, with particular reference to the figures of the enclosed drawings, wherein:
figure 1 shows an operative block diagram of the system for controlling healthiness in food chain and/or environment (toxicity indexes) according to the present invention; and
figure 2 shows a control chart of water integral toxicity during a step of the food chain critical for contamination of metals.

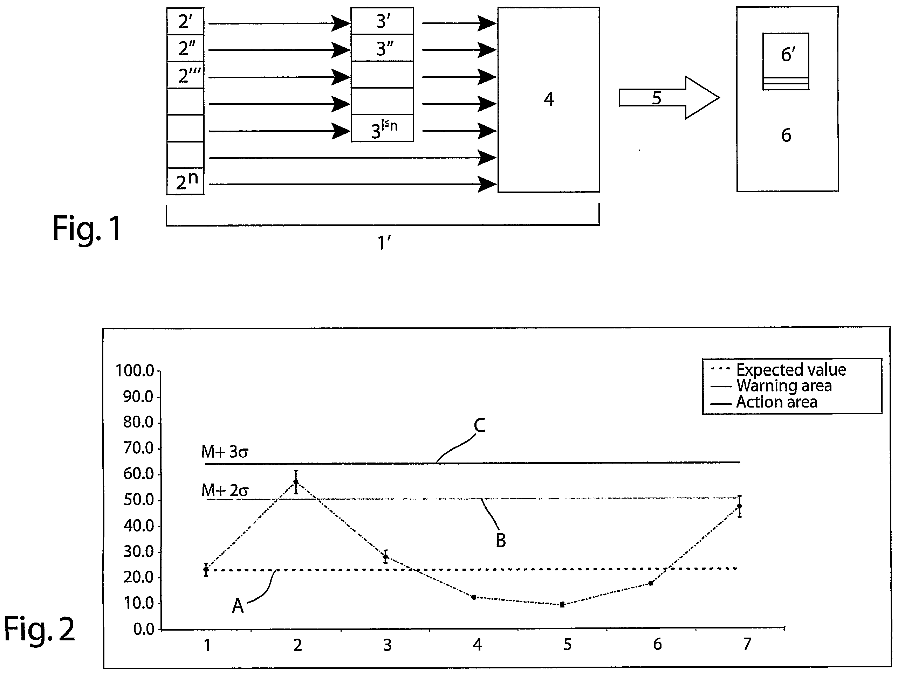

Making reference to figure 1, it is possible observing an integrated system 1 for diagnosis and monitoring a productive food chain and eco-toxicity and bio-restoration, that can be also applied for self-control in a toxicological field of the food chain, starting from primary production, by early identification, control, monitoring and managing of anomalous variations of an integrated grid of toxicity indexes (including bio-markers of total exposition or of efficient dose, non specific, specific, secondary and support indexes) with respect to the variation interval which is accepted as normal according to the present invention, comprising a detection unit 1' and a data processing and managing unit 6. Said detection unit 1' comprises a plurality (n) of probes 2', 2", ..., 2ⁿ, a plurality (I) of electronic boards for detection/pre-processing/sending the electric signal of the relevant connected probe (3', 3", ..., 3ⁿ, wherein 1≤n), a data collecting module 4 suitable to store detections and calibration set before of said probes 2', 2", ..., 2ⁿ.

More specifically, said probes 2', 2", ..., 2ⁿ, can be provided with a plurality m of biologic means (to realize biosensors), wherein 0≤m≤n, provided in a thermostat environment, preferably with liquid fluid, or in a climatised environment. Each one of said probes 2', 2", ..., 2ⁿ can be independently connected with a relevant electronic board detecting signals 3', 3", ..., 3ⁿ. said electronic boards 3', 3", ..., 3ⁿ, are then connected with said data collecting module 4, connecting n signals (I of which collected through said electronic boards 3) at the same time (in parallel) to send them, by a serial interface 5, to said data processing unit 6, possibly comprised of a computer, provided with user interface means, such as a monitor 6' or like, wherein control chart can be displayed as early hazard managing means.

Signals generated by probes 2', 2", ..., 2ⁿ are recorded by data processing unit 6, providing also to transform information according to the "data in - graphic out" mode. In other words, data processing unit 6 acquires signals (current, voltage, resistance, analogical/digital signals) continuously detected by different probes 2', 2", ..., 2ⁿ, either directly and pre-processed by said transduction electronic boards 3', 3", ..., 3^{l}, and collected by said data collecting module 4. Monitor 6' shows run of signal arriving from said n probes 2', 2", ..., 2ⁿ processed in n control charts, that will be better described in the following.

Data processing unit 6 is possibly provided with a warning device that can emit a sound or luminous signal, to warn an operator about good operation of samples and on process control, in case one or more values pass the relevant control thresholds.

In case, serial interface 5 can be suitable to transmit data, even wireless, by intranet or internet connection, so that data can be remote monitored and processed (or displayed) in real time for tracking and managing hazards.

Said probes 2', 2", ..., 2ⁿ can be coupled with biological systems to realize biosensors. In fact, integrated system 1 has flexible parameters and can be suitable to the specific problems identified in a certain food chain and/or situation. Particularly, it is possible selecting a specific range of parameters for a set problem of analyses to be carried out.

More specifically, it is possible grouping probes 2', 2", ..., 2ⁿ that are aimed to valuation of toxicity, for illustrative but not limitative purposes, preferably in 4 groups on the basis of parameters that they can detect, such as:
- a first group of parameters, comprised of electrochemical/optical probes coupled with microorganisms (whole or part of them, or modified), also facultative aerobe (e.g. leaven cells) or obliged (e.g. human cell lines) or of the photosynthetic type (e.g. algae or chloroplasts), or chemo-luminescent (e.g. chemo-luminescent bacteria or enzymes), for evaluating integral toxicity index by measurement of cellular respiration and/or photosynthetic activity;
- a second group of parameters comprised of electrochemical probes coupled with general indicators of oxidoreductase enzymatic activity such as antioxidant cellular enzymes as superoxidodismutase, glutathione peroxidase and/or glucose metabolism such as glucose oxidase, dehydrogenase lactate;
- a third group of parameters comprised of probes for measurement of secondary indexes supporting information obtained by said first and said second group of probes, for example glucose (index of energetic metabolism), Biologic Oxygen Demand (BOD), excreted enzymes (electrochemical probe coupled with urease enzyme, applicable in a specific way for heavy metals), indicators of presence of active concentrations of pesticides (e.g. cholinesterase, tirosinase, photosynthetic activity) and antibiotic α-lactamic (α-lactamase), and presence of ions, such as nitrate and ammonium (ion-selective electrodes);
- a fourth group of parameters comprised of support probes measuring: pH, temperature, conductivity, redox potential, O₂/CO₂ and chemical oxygen demand (COD).

In this way, essential information of the integrated system 1 can be for example comprised of:
- integral toxicity on cells as general indicator of healthiness;
- anti-oxidant protective enzymes;
- pH, conductivity, redox homeostase and temperature.

It is even possible integrating immunosensors, probes and DNA in the system.

These essential information are powered and enriched by specific information provided by integration with other parameters, selected on the basis of application and possible problems and critical parameters individuated. Data processing unit 6 also permits a statistic approach and multivaried analyses for evaluation of toxicity indexes. Said multivaried analyses can be carried out on data detected by probes 2', 2", ..., 2ⁿ even in real time and permits use of system 1 and of result obtained also by not specialized operators. According to another embodiment, said data processing unit 6 can be also based on neural networks, permitting a systematic learning while detecting and checking adequacy of productive process under examination which is improved with time.

As it can be noted, integrated system 1 permits of:
a. identifying critical points for managing possible toxicological hazard during the productive process, starting from environment,
b. discriminating among batches along food chain, from raw material to treatment and finished product;
c. generating warning/alarm signals for quick individuation and managing of hazards.

Therefore, integrated system here described is a system and method of quality improvement based on control during production finalized to verifying respect during productive process of the design set standards.

Integrated system 1, and particularly processing unit 6, permits, as already said, analyses, substantially in real time, of data detected by probes 2', 2", ..., 2ⁿ in order to permit a quick interpretation of results, either single results or the whole results.

A suitable method thus manages all the above data, maintaining raw data in order to ensure their tracking. Complexity of information that can be obtained is managed in such a way to:
- use necessary information (e.g. integral toxicity) as healthiness general index, specific information (e.g. presence of pesticides at an active concentration), and redundant (e.g. respiration and glucose);
- identifying additional, compensative or synergic effects (e.g. pH effect and temperature on enzymes);
- evaluation of feedback noise and correlation.

An operator, even not skilled in the field, by having a suitable system according to the invention can quickly be aware, by a signal emitted by said alarm device, if a process is out of control as far as one or more of the n chosen parameters is concerned and correlated with safety of the specific production.

Criterion for emitting signal is based on warning limit (2σ) or intervention limit (3σ) approach while whole data processing and interpretation can be obtained by a multivaried analyses carried out, as already said, in real time.

System 1 is provided with a software permitting automatically building quick reading graphs for each parameter. Particularly, integrated system 1 shows control charts on the relevant monitor 6', as the one shown in figure 2, which is a control chart for integral toxicity of water in a CCP for metal contamination.

Ordinates in said control chart show percentage of toxicity, while abscissas show succession of measurements on different water batches.

To realize chart, water samples are contaminated with different amounts of metallic ion (Platinum) (from 200 to 650 mg for ml of water). Point 2 (second measure of the succession) is the warning point.

Control charts are realized by system 1 for each toxicological parameters measured by relevant probes 2', 2", ..., 2ⁿ selected for each critical step of every productive process.

At the beginning, when expected value (reference value of the productive practice) shown from line A, value M, for each parameter in the food chain critical point is not well known, it must be detected by acquisition for realizing control chart. Thus, expected values, line A, and warning limits, line B and intervention values, line C, must be derivated by operation of system 1.

Line A is calculated as average value, while warning limits and intervention limits are calculated in function of value of line A (value of M ordinate) and of variability (σ) which is considered as physiological for that production or between batches or within environment, i.e. in the present embodiment, average square difference.

Particularly, warning limit shown in figure by line B is calculated as M±2σ and intervention limit as M±3σ, being σ a variability or physiological variability range for that specific production or environment. Warning and intervention limits indicate range within which the process is under control.

If available, recognized and updated, it is possible taking as expected value (line A) a reference value, and monitoring displacement of samples with respect to the same. In other words, line A is not calculated by system 1 as average of measurements, but it is already known.

Therefore robustness and accuracy of control chart increases with time by the continuous contribution of food chain data; it also permits evaluating impact of changes in food chain or improving or corrective intervention, that can bring for example to reduction of σ; it further permits evaluating positiveness and efficiency of corrective actions.

In order to monitor proper maintenance of performances of sample while time passes (standard error of method), control chart also shows standard deviation of the measures (number of repetitions corresponding to number of electrodes for that sample).

For biosensors, performance of sample is monitored by a control chart on the not-exposed side: an acoustic/sound signal emitted by said alarm device warns operator if an anomalous deviation from reference values occurs.

Process is considered out of the control for every critical point, on the basis of set operative procedures.

For example, on the basis of the specific case, it can be required a corrective action (for example taking out/decontaminating or sending to official analyses for deepening the matter) when for example a detection crosses intervention line C, two consecutive detections passed warning line B, or when a sequence of continuously growing detection sequence is recorded. Control limits are usually updated on the basis of a method modification.

Besides continued control, information provided by said system can be also used for detecting alterations of conformity parameters of environmental or food samples.

Integrated system 1 and early data processing and managing method of hazards described in the above can be particularly applied on environment and food matrixes, such as, for example, water, milk, wine, fruit juices, waste waters. System 1 is versatile and adaptable to the specific food chain and CCP individuated, intervening in at least 4 of the 7 HACCP principles, as described in the following table.

| | | |
|---|---|---|
| 1 | Analyses of hazards for each step of productive process | Support to identification of hazards in the productive chain |
| 2 | Identification of CCP to be monitored | |
| 3 | Definition of critical limits for each CCP | In view of lack of set reference values, both expected values and warning and intervention limits are obtained/set *in situ* by acquisition during the productive life or the factory. Therefore, sturdiness and accuracy of said values increases while time passes, with acquisition of data concerning productive life |
| 4 | Definition of monitoring procedures | i) *in situ* method permits continuous data recording, |
| | | ii) wireless and intranet/internet coordination without reduction of performances, among different places in different geographical areas and operative central unit |
| | | iii) "data in - graphic out" approach permits a simple interpretation by not specialized personnel for ready intervention and with corrective actions |
| 5 | Definition of corrective actions | |
| 6 | Definition of procedures for checking conformity of HACCP system with HACCP plane | Control charts verify positiveness/efficiency of corrective actions |
| 7 | Preparation of system for managing documents | |

More in general, four application sectors can be individuated for illustrative but not limitative purposes:
- "process" samples (control charts): food and feed production chains, depuration processes (e.g. waste waters; process wastes); agro-zootechnical activity impact;
- screening "punctiform" samples: alteration and reversibility of conformity parameters for food and environmental samples; evaluation of impact of diet and/or of its components in the zootechnical breeding with minimum in vivo invasivity (biological fluids, including milk);
- applied and basic research. Some examples of this possible application could be: study of contamination sources; study of protective factors and risk/benefit evaluation; study of chemical affinity and toxicity of real matrixes; study of enzymatic and mitochondrial targets and individuation of values of maximum concentrations to be recommended as reference in the check charts;
- interface between production (acquisition of toxicity indexes in the real productive realty) and research, control and industry.

An advantage of the present invention is that system permits definition of validation parameters for each test.

The present invention has been described for illustrative but not limitative purposes, according to its preferred embodiments, but it is to be understood that modifications and/or changes can be introduced by those skilled in the art without departing from the scope as defined in the enclosed claims.

## Claims

1. System (1) for environmental diagnostics and monitoring and toxicological self-monitoring and traceability in the food chain, including primary production, comprising:
a detecting unit (1'), having a plurality of independently selectable probes (2', 2"..., 2n),
a data processing unit (6), connectable to a data collecting module (4), for acquiring said detected data of said probes (2', 2"..., 2n), provided with user interface means (6'),
wherein
said detecting unit (1') is provided with a plurality m of biological means, wherein 0≤m≤n,
each one of said n probes is suitable to detect a respective parameter relevant to quality and/or safety, thereby generating a suitable signal,
said data collecting module (4) is connected to said probes (2', 2"..., 2n) and suitable to store the so detected data,
said data processing unit (6) is capable of simultaneously controlling charting the signal of the said probes, identifying and determining concurrent anomalies of said parameters in control charts and/or other diagrams, and providing by said interface means (6') a warning indication based on at least one alert threshold or at least one intervention threshold from the parameters detected by said selected probes (2', 2"..., 2n), through the comparison of the detected data of said probes (2', 2"..., 2n) with external or self-educated thresholds, in one or more Critical Control Points (CCP) and/or points of particular attention of a food chain and/or a environmental compartment, and
said parameters are of biological and/or chemical and/or physical type,
said system (1) being **characterized**
**in that** it comprises a plurality of electronic boards (3', 3"..., 3n), each one connected to one respective probe (2', 2"..., 2n) and to said data collecting module (4), and suitable for carrying out a preprocessing of the signal of the respective probe (2', 2"..., 2n),
**in that** said probes (2', 2"..., 2n) and the relevant electronic boards (3', 3"..., 3n) are connected in parallel, so that said system (1) is suitable to carry out diagnostic, environmental monitoring and toxicological self-monitoring and traceability along different stations in the food chain, including primary production, by early identification, control, monitoring and managing of anomalous variations of an integrated grid of indexes, with respect to a variation range which is considered normal, and
**in that** said data processing unit (6) is configured to carry out the following steps for realizing control charts and/or other diagrams:
- carrying out a plurality of measures of one or more parameters by means of the corresponding probes (2', 2"..., 2n);
- setting an expected measurement value (M) of each one of said parameters;
- calculating a variability interval or physiological variation (σ) for each one of said plurality of measures for each one of said parameters;
- calculating, for one or more parameters, at least one alert indication threshold as a function of said physiological variation (σ);
- calculating for one or more parameters at least one intervention indication threshold as a function of said physiological variation (σ), suitable to provide an intervention indication for controlling food healthiness and/or toxicity indexes in the food chain and/or in the environment; and
- carry out a statistical analysis and multivariate analysis of the values of data detected by said probes (2', 2"..., 2n) to assess said integrated indexes;
wherein
the expected value of a parameter is equal to the average of the measurements carried out, said physiological variation (σ) being the variability interval of said measurements of said parameters and robustness and precision of said control chart increasing consistency of said expected value (M) and of said physiological variation (σ) following the input of data; and
said alert indication threshold is equal to twice the physiological variation (2σ) and said intervention indication threshold is equal to three times the physiological variation (3σ).

2. System (1) according to claim 1, **characterized in that** said detection unit (1') is supplied by electric supply, or by a battery, or by eco-compatible sources, such as solar energy.

3. System (1) according to one of the preceding claims, **characterized in that** said probes (2', 2"..., 2n) are of the electric/electrochemical/optic type, preferably of the type coupled with microorganisms, said organisms being whole or part of microorganism, facultative aerobe, such as leaven cells, or obliged, such as human cellular lines or even of the photosynthetic or chemo-luminescent type for valuation of the integral toxicity index by measure of cellular respiration and/or photosynthetic activity and/or of chemo-luminescence and/or of the type coupled with general indicators of oxidoreductase enzymatic activity and/or DNA and/or immunosensors.

4. System (1) according to one of the preceding claims, **characterized in that** said signal generated by said probes (2', 2"..., 2n) are of analogical and/or digital type, said probes (2', 2"..., 2n) being suitable to measure further indexes, such as glucose and/or the oxygen biological demand (BOD) and/or secretion enzymes and/or active concentrations of pesticides and antibiotic and/or presence of ions.

5. System (1) according to one of the preceding claims, **characterized in that** said probes (2', 2"..., 2n) are suitable to measuring pH and/or temperature and/or conductivity and/or redox potential and/or O₂/CO₂ and/or Chemical Oxygen Demand (COD) and/or
said probes (2', 2"..., 2n) are modular and are suitable to the specific problems identified in a specific chain and/or measure situation.

6. System (1) according to one of the preceding claims, **characterized in that** said probes (2', 2"..., 2n) are maintained in a liquid fluid thermostated environment or in a climatised environment, at a controlled temperature, with monitoring and registration of the operating temperature.

7. System (1) according to one of the preceding claims, **characterized in that** said data collecting module (4) is connected with said data processing unit (6) by a serial interface (5) for intranet and/or by wireless connection and/or by intranet network and/or by internet network, to transmit data from said detections, said data processing unit (6) is connected to a plurality of said data collecting module (4), said system (1) comprises an acoustic and/or optic warning device suitable to emit a signal in case at least one detection of one of said probes (2', 2"..., 2n) goes beyond the respective warning or alert threshold.

8. System (1) according to one of the preceding claims, **characterized in that** said data processing unit (6) is based on neural networks.

9. System (1) according to one of the preceding claims, **characterized in that** it comprises a menu, by which the parameters and the respective probes (2', 2"..., 2n) are preselected or preselectable for specific foods or for specific critical control points (CCP) and/or points of particular attention in the food chain and/or in environment, and
**in that** said interface means comprises a monitor (6').

10. System (1) according to one of the preceding claims, **characterized in that** it communicates by wireless transceiving means or by intranet or internet network, with further systems (1), each one of said systems (1) being applied in different environmental compartments and/or different Critical Control Points (CCP) and/or points of particular attention along the different branches of a food chain or environment, including primary production.

11. System according to any one of the preceding claims, **characterized in that** the expected value of the parameters is preset and/or established by the control charting practice in the specific application field and/or derived by the measurements.

12. System (1) according to anyone of the preceding claims, **characterized in that** said said data processing unit (6) is configured to carry out the following further steps:
- generating an alarm signal in case of detection of at least one parameter passing two consecutive times the respective warning indication threshold and/or at least once the relevant intervention indication threshold and
- displaying, on said interface means, one control chart for each detected parameter, suitable to display the respective detected data, the expected value (M), at least one of said warning threshold, at least one said intervention indication threshold, and measurement errors, to detect simultaneous anomalies in parallel control charts, and integrated indexes.

## Patentansprüche

1. System (1) für Umgebungsdiagnostik und -überwachung und toxikologische Selbstüberwachung und Rückverfolgbarkeit in der Nahrungsmittelkette, Primärproduktion beinhaltend, umfassend:
eine Detektionseinheit (1'), die eine Vielzahl von unabhängig auswählbaren Sonden (2', 2"..., 2n) aufweist;
eine Datenverarbeitungseinheit (6), die mit einem Datensammelmodul (4) verbindbar ist, zum Erfassen der detektierten Daten der Sonden (2', 2"..., 2n), mit Benutzerschnittstellenmitteln (6') bereitgestellt;
wobei
die Detektionseinheit (1') mit einer Vielzahl m von biologischen Mitteln bereitgestellt wird, wobei 0≤m≤n,
jede der n Sonden geeignet ist, einen jeweiligen Parameter zu detektieren, der relevant für Qualität und/oder Sicherheit ist, wodurch ein geeignetes Signal erzeugt wird,
das Datensammelmodul (4) mit den Sonden (2', 2"..., 2n) verbunden ist, und geeignet, um die so detektierten Daten zu speichern,
die Datenverarbeitungseinheit (6) in der Lage ist, gleichzeitig das Kartieren des Signals der Sonden zu steuern, übereinstimmende Anomalien der Parameter in Kontrollkarten und/oder anderen Diagrammen zu identifizieren und zu bestimmen, und durch die Schnittstellenmittel (6') einen Warnhinweis basierend auf mindestens einem Alarmgrenzwert oder mindestens einem Interventionsgrenzwert von den Parametern bereitzustellen, die durch die ausgewählten Sonden (2', 2"..., 2n) detektiert worden sind, durch das Vergleichen der durch die Sonden (2', 2"..., 2n) detektierten Daten mit externen oder autodidaktischen Grenzwerten in einem oder mehreren kritischen Kontrollpunkten (CCP) und/oder Punkten mit besonderer Aufmerksamkeit einer Nahrungsmittelkette und/oder eines Umgebungskompartiments, und
die Parameter biologischer und/oder chemischer und/oder physikalischer Art sind,
wobei das System (1) **dadurch gekennzeichnet ist,**
**dass** es eine Vielzahl von Elektronikkarten (3', 3"..., 3n) umfasst, von denen jede mit einer jeweiligen Sonde (2', 2"..., 2n) und mit dem Datensammelmodul (4) verbunden ist, und geeignet ist, eine Verarbeitung des Signals der jeweiligen Sonde (2', 2"..., 2n) durchzuführen,
dadurch, dass die Sonden (2', 2"..., 2n) und die relevanten Elektronikkarten (3', 3"..., 3n) parallel verbunden sind, sodass das System (1) geeignet ist, Diagnose, Umgebungsüberwachung und toxikologische Selbstüberwachung und Rückverfolgbarkeit entlang unterschiedlicher Stationen in der Nahrungsmittelkette, Primärproduktion beinhaltend, durch frühe Identifizierung, Steuerung, Überwachung und Regeln anormaler Variationen eines integrierten Rasters an Indizes in Bezug auf einen Variationsbereich durchzuführen, der als normal angenommen wird, und
dadurch, dass die Datenverarbeitungseinheit (6) konfiguriert ist, um die folgenden Schritte durchzuführen, um Kontrollkarten und/oder andere Diagramme zu realisieren:
- Durchführen einer Vielzahl von Messungen von einem oder mehreren Parametern anhand der entsprechenden Sonden (2', 2"..., 2n);
- Einstellen eines erwarteten Messwerts (M) von jedem der Parameter;
- Berechnen eines Variabilitätsintervalls oder einer physiologischen Variation (*σ*) für jede der Vielzahl von Messungen für jeden der Parameter;
- Berechnen mindestens eines Alarmhinweisgrenzwertes für einen oder mehrere Parameter in Abhängigkeit von der physiologischen Variation (*σ*);
- Berechnen mindesten eines Interventionshinweisgrenzwertes für einen oder mehrere Parameter in Abhängigkeit von der physiologischen Variation (*σ*), geeignet, um einen Interventionshinweis zum Kontrollieren der Gesundheit der Nahrungsmittel und/oder von Toxizitätsindizes in der Nahrungsmittelkette und/oder in der Umgebung bereitzustellen; und
- Durchführen einer statistischen Analyse und einer multivariaten Analyse der von den Sonden (2', 2"..., 2n) detektieren Datenwerte zum Bewerten der integrierten Indizes;
wobei
der erwartete Wert eines Parameters gleich dem Mittelwert der durchgeführten Messungen ist, wobei die physiologische Variation (*σ*) das Variabilitätsintervall der Messungen der Parameter ist und Robustheit und Präzision der Kontrollkarte die Konsistenz des erwarteten Wertes (M) und der physiologischen Variation (*σ*) nach der Dateneingabe steigert; und
der Alarmhinweisgrenzwert gleich zweimal der physiologischen Variation (2*σ*) ist und der Interventionshinweisgrenzwert gleich dreimal der physiologischen Variation (3*σ*) ist.

2. System (1) nach Anspruch 1, **dadurch gekennzeichnet, dass** die Detektionseinheit (1') durch Stromversorgung, oder durch eine Batterie, oder durch ökokompatible Quellen, wie Sonnenenergie, versorgt wird.

3. System (1) nach einem der vorstehenden Ansprüche, **dadurch gekennzeichnet, dass** die Sonden (2', 2"..., 2n) der elektrischen/elektrochemischen/optischen Art sind, vorzugsweise in der Art mit Mikroorganismen gekoppelt, wobei die Organismen ganz oder ein Teil von, fakultativ aeroben, Mikroorganismen, wie Treibmittelzellen sind, oder obligatorisch, wie menschliche Zelllinien oder selbst in der photosynthetischen oder chemilumineszenten Art, zur Bewertung des integralen Toxizitätsindex durch Messen von zellulärer Respiration und/oder photosynthetischer Aktivität und/oder Chemilumineszenz und/oder in der Art mit allgemeinen Indikatoren für Oxidoreduktase enzymatische Aktivität und/oder DNA und/oder Immunsensoren gekoppelt.

4. System (1) nach einem der vorstehenden Ansprüche, **dadurch gekennzeichnet, dass** das durch die Sonden (2', 2"..., 2n) erzeugte Signal der analogen und/oder digitalen Art ist, wobei die Sonden (2', 2"..., 2n) geeignet sind, weitere Indizes, wie Glukose- und/oder biochemischen Sauerstoffbedarf (BOD) und/oder Sekretionsenzyme und/oder aktive Konzentrationen von Pestiziden und Antibiotikum und/oder eine Präsenz von Ionen zu messen.

5. System (1) nach einem der vorstehenden Ansprüche, **dadurch gekennzeichnet, dass** die Sonden (2', 2"..., 2n) geeignet sind zum Messen von pH und/oder Temperatur und/oder Leitfähigkeit und/oder Redoxpotential und/oder O₂/CO₂ und/oder chemischen Sauerstoffbedarf (COD) und/oder
die Sonden (2', 2"..., 2n) modular sind und geeignet für die speziellen Probleme sind, die in einer speziellen Kette und/oder Messsituation identifiziert werden.

6. System (1) nach einem der vorstehenden Ansprüche, **dadurch gekennzeichnet, dass** die Sonden (2', 2"..., 2n) in einer flüssigen fluid-thermostatierten Umgebung oder in einer klimatisierten Umgebung auf einer kontrollierten Temperatur, mit Überwachung und Registrierung der Betriebstemperatur gehalten werden.

7. System (1) nach einem der vorstehenden Ansprüche, **dadurch gekennzeichnet, dass** das Datensammelmodul (4) durch eine serielle Schnittstelle (5) für Intranet und/oder durch eine drahtlose Verbindung und/oder durch Intranet-Netzwerk und/oder durch Internet-Netzwerk mit der Datenverarbeitungseinheit (6) verbunden ist, um Daten von den Detektionen zu übertragen, die Datenverarbeitungseinheit (6) mit einer Vielzahl eines solchen Datensammelmoduls (4) verbunden ist, das System (1) eine akustische und/oder optische Warnvorrichtung umfasst, die geeignet ist, ein Signal im dem Falle auszugeben wenn mindestens eine Detektion von einer der Sonden (2', 2"..., 2n) über den Warnungs- oder Alarmgrenzwert hinausgeht.

8. System (1) nach einem der vorstehenden Ansprüche, **dadurch gekennzeichnet, dass** die Datenverarbeitungseinheit (6) auf neuronalen Netzwerken basiert.

9. System (1) nach einem der vorstehenden Ansprüche, **dadurch gekennzeichnet, dass** es ein Menü umfasst, durch das die Parameter und die jeweiligen Sonden (2', 2"..., 2n) vorgewählt werden oder für spezielle Nahrungsmittel oder für spezielle kritische Kontrollpunkte (CCP) und/oder Punkte mit besonderer Aufmerksamkeit in der Nahrungsmittelkette und/oder in der Umgebung vorwählbar sind, und
dadurch, dass das Schnittstellenmittel einen Monitor (6') umfasst.

10. System (1) nach einem der vorstehenden Ansprüche, **dadurch gekennzeichnet, dass** es durch drahtlose Sende-/Empfangsmittel oder durch Intranet- oder Internet-Netzwerk mit weiteren Systemen (1) kommuniziert, wobei jedes der Systeme (1) in verschiedenen Umgebungskompartimenten und/oder verschiedenen kritischen Kontrollpunkten (CCP) und/oder Punkten mit besonderer Aufmerksamkeit entlang der verschiedenen Äste einer Nahrungsmittelkette oder Umgebung, Primärproduktion beinhaltend, angewendet wird.

11. System nach einem der vorstehenden Ansprüche, **dadurch gekennzeichnet, dass** der erwartete Wert der Parameter durch die Kontrollkartenpraxis in dem speziellen Anwendungsfeld voreingestellt und/oder festgelegt wird oder von den Messungen abgeleitet wird.

12. System (1) nach einem der vorstehenden Ansprüche, **dadurch gekennzeichnet, dass** die Datenverarbeitungseinheit (6) konfiguriert ist, um die folgenden weiteren Schritte durchzuführen:
- Erzeugen eines Alarmsignals im Falle einer Detektion von mindestens einem Parameter, der zwei Mal hintereinander den jeweiligen Alarmhinweisgrenzwert und/oder einmal den relevanten Interventionshinweisgrenzwert überschreitet, und
- Anzeigen, auf dem Schnittstellenmittel, einer Kontrollkarte für jeden detektierten Parameter, geeignet, um die jeweiligen detektierten Daten, den erwarteten Wert (M), mindestens einen des Warngrenzwerts, mindestens einen des Interventionshinweisgrenzwerts, und Messfehler anzuzeigen, um gleichzeitige Anomalien in parallelen Kontrollkarten und integrierten Indizes zu detektieren.

## Revendications

1. Système (1) pour le diagnostic et la surveillance de l'environnement et l'autosurveillance toxicologique et la traçabilité de la chaîne alimentaire, y compris la production primaire, comprenant :
une unité de détection (1'), ayant une pluralité de sondes pouvant être sélectionnées indépendamment (2', 2", ..., 2n),
une unité de traitement de données (6), pouvant être reliée à un module collecteur de données (4), pour acquérir lesdites données détectées desdites sondes (2', 2", ..., 2n), munie d'un moyen d'interface utilisateur (6'),
dans lequel
ladite unité de détection (1') est munie d'une pluralité m de moyens biologiques, dans lequel 0≤m≤n,
chacune desdites n sondes est appropriée pour détecter un paramètre respectif pertinent pour la qualité et/ou la sécurité, produisant de ce fait un signal approprié,
ledit module collecteur de données (4) est relié auxdites sondes (2', 2", ..., 2n) et approprié pour stocker les données ainsi détectées,
ladite unité de traitement de données (6) est susceptible de commander simultanément la représentation sous forme graphique du signal desdites sondes, l'identification et la détermination d'anomalies concourantes desdits paramètres dans des diagrammes de commande et/ou d'autres schémas, et de fournir par ledit moyen d'interface (6') une indication d'avertissement basée sur au moins un seuil alerte ou au moins un seuil d'intervention à partir des paramètres détectés par lesdites sondes sélectionnées (2', 2", ..., 2n), par la comparaison des données détectées desdites sondes (2', 2", ..., 2n) à des seuils externes ou autodidactes, dans un ou plusieurs Points Critiques de Maîtrise (CCP) et/ou points d'attention particulière d'une chaîne alimentaire et/ou d'un compartiment environnemental, et
lesdits paramètres sont de type biologique et/ou chimique et/ou physique,
ledit système (1) étant **caractérisé**
**en ce qu'**il comprend une pluralité de cartes électroniques (3', 3", ..., 3n), chacune reliée à une sonde particulière respective (2', 2", ..., 2n) et audit module collecteur de données (4) et appropriées pour exécuter un prétraitement du signal de la sonde respective (2', 2", ..., 2n),
**en ce que** lesdites sondes (2', 2", ..., 2n) et les cartes électroniques appropriées (3', 3", ..., 3n) sont reliées en parallèle, de sorte que ledit système (1) est approprié pour exécuter un diagnostic, une surveillance environnementale et une autosurveillance toxicologique et une traçabilité le long de différentes stations dans la chaîne alimentaire, y compris la production primaire, par une identification, une maîtrise, une surveillance et une gestion précoces de variations anormales d'une grille d'indices intégrés, par rapport à une plage de variations que l'on considère normale, et
**en ce que** ladite unité de traitement de données (6) est configurée pour exécuter les étapes suivantes pour réaliser des diagrammes témoin et/ou d'autres schémas :
- exécution d'une pluralité de mesures d'un ou plusieurs paramètres au moyen des sondes correspondantes (2', 2", ..., 2n) ;
- fixation d'une valeur de mesure attendue (M) de chacun desdits paramètres ;
- calcul d'un intervalle de variabilité ou une variation physiologique (σ) pour chacune de ladite pluralité de mesures pour chacun desdits paramètres ;
- calcul, pour un ou plusieurs paramètres, d'au moins un seuil d'indication alerte comme une fonction de ladite variation physiologique (σ) ;
- calcul pour un ou plusieurs paramètres d'au moins un seuil d'indication d'intervention comme une fonction de ladite variation physiologique (σ), approprié fournir une indication d'intervention pour maîtriser des indices de salubrité et/ou de toxicité alimentaire dans la chaîne alimentaire et/ou dans l'environnement ; et
- exécution d'une analyse statistique et d'une analyse à variables multiples des valeurs de données détectées par lesdites sondes (2', 2", ..., 2n) pour évaluer lesdits indices intégrés ;
dans lequel
la valeur attendue d'un paramètre est égale à la moyenne des mesures effectuées, ladite variation physiologique (σ) étant l'intervalle de variabilité desdites mesures desdits paramètres et la robustesse et la précision dudit diagramme témoin augmentant la cohérence de ladite valeur attendue (M) et de ladite variation physiologique (σ) après l'entrée de données ; et
ledit seuil d'indication d'alerte est égal à deux fois la variation physiologique (2σ) et ledit seuil d'indication d'intervention est égal à trois fois la variation physiologique (3σ).

2. Système (1) selon la revendication 1, **caractérisé en ce que** ladite unité de détection (1') est alimentée par une alimentation électrique, ou par batterie, ou par des sources éco-compatibles, comme de l'énergie solaire.

3. Système (1) selon l'une des revendications précédentes, **caractérisé en ce que** lesdites sondes (2', 2", ..., 2n) sont du type électrique/électrochimique/optique, de préférence du type couplé à des micro-organismes, lesdits organismes constituant tout ou partie de micro-organisme, de manière facultative un aérobie, comme des cellules de levain, ou obligé, comme des lignées cellulaires humaines ou même du type photosynthétique ou chimioluminescent pour l'estimation de l'indice de toxicité intégrale par la mesure de la respiration cellulaire et/ou de l'activité photosynthétique et/ou de la chimioluminescence et/ou du type couplé à des indicateurs globaux de l'activité enzymatique d'oxydo-réductase et/ou l'ADN et/ou des immunocapteurs.

4. Système (1) selon l'une des revendications précédentes, **caractérisé en ce que** ledit signal produit par lesdites sondes (2', 2", ..., 2n) sont de type analogique et/ou numérique, lesdites sondes (2', 2", ..., 2n) étant appropriées pour mesurer des indices supplémentaires, comme le glucose et/ou la Demande Biologique en Oxygène (DBO) et/ou des enzymes de sécrétion et/ou des concentrations actives de pesticides et d'antibiotique et/ou la présence d'ions.

5. Système (1) selon l'une des revendications précédentes, **caractérisé en ce que** lesdites sondes (2', 2", ..., 2n) sont appropriées pour la mesure du pH et/ou de la température et/ou de la conductivité et/ou du potentiel d'oxydo-réduction et/ou du rapport O₂/CO₂ et/ou de la Demande Chimique en Oxygène (DCO) et/ou
lesdites sondes (2', 2", ..., 2n) sont modulaires et sont appropriées aux problèmes spécifiques identifiés dans une situation de mesure et/ou une chaîne spécifique.

6. Système (1) selon l'une des revendications précédentes, **caractérisé en ce que** lesdites sondes (2', 2", ..., 2n) sont maintenues dans un environnement thermostaté fluide liquide ou dans un environnement climatisé, à une température maîtrisée, avec surveillance et enregistrement de la température de fonctionnement.

7. Système (1) selon l'une des revendications précédentes, **caractérisé en ce que** ledit module collecteur de données (4) est relié à ladite unité de traitement de données (6) par une interface série (5) pour l'intranet et/ou par une connexion sans fil et/ou par réseau intranet et/ou par réseau Internet, pour transmettre des données à partir desdites détections, ladite unité de traitement de données (6) est reliée à une pluralité de dits modules collecteurs de données (4), ledit système (1) comprend un dispositif d'avertissement acoustique et/ou optique approprié pour émettre un signal dans le cas où au moins une détection d'une desdites sondes (2', 2", ..., 2n) va au-delà du seuil respectif d'avertissement ou d'alerte.

8. Système (1) selon l'une des revendications précédentes, **caractérisé en ce que** ladite unité de traitement de données (6) est basée sur des réseaux de neurones artificiels.

9. Système (1) selon l'une des revendications précédentes, **caractérisé en ce qu'**il comprend un menu, par lequel les paramètres et les sondes respectives (2', 2", ..., 2n) sont présélectionnés ou peuvent être présélectionnés pour des produits alimentaires spécifiques ou pour des Points Critiques de Maîtrise (CCP) et/ou des points d'attention particulière dans la chaîne alimentaire et/ou dans l'environnement, et
**en ce que** ledit moyen d'interface comprend un dispositif de contrôle (6').

10. Système (1) selon l'une des revendications précédentes, **caractérisé en ce qu'**il communique par un moyen d'émission-réception sans fil ou par réseau intranet ou Internet, avec des systèmes supplémentaires (1), chacun desdits systèmes (1) étant appliqué dans des compartiments environnementaux différents et/ou des Points Critiques de Maîtrise différents (CCP) et/ou des points d'attention particulière le long des différentes branches d'une chaîne alimentaire ou d'un environnement, y compris la production primaire.

11. Système selon l'une quelconque des revendications précédentes, **caractérisé en ce que** la valeur attendue des paramètres est définie à l'avance et/ou établie par la pratique de représentation témoin sous forme graphique dans le champ spécifique d'application et/ou obtenue par les mesures.

12. Système (1) selon l'une quelconque des revendications précédentes, **caractérisé en ce que** ladite unité de traitement de données (6) est configurée pour exécuter les étapes supplémentaires suivantes :
- production d'un signal d'alarme en cas de détection d'au moins un paramètre passant deux fois consécutives le seuil d'indication d'avertissement respectif et/ou au moins une fois le seuil d'indication concerné d'intervention, et
- affichage, sur ledit moyen d'interface, d'un diagramme témoin pour chaque paramètre détecté, approprié pour afficher les données détectées respectives, la valeur attendue (M), au moins un dudit seuil d'avertissement, au moins un dudit seuil d'indication d'intervention et des erreurs de mesure, pour détecter des anomalies simultanées dans des diagrammes témoin parallèles et des indices intégrés.
